# EUROPEAN PATENT APPLICATION

(11) **EP 1 825 844 A1**
(43) Date of publication of application: **29.08.2007**
(21) Application number: 05819591.8
(22) Date of filing: 16.12.2005
(51) Int. Cl.: A61K 8/97

(54) **SKIN WHITENING PREPARATION FOR EXTERNAL USE, WHITENING PREPARATION, WHITENING METHOD AND METHOD OF PRODUCING SKIN WHITENING PREPARATION FOR EXTERNAL USE**

(30) Priority: 17.12.2004 JP 2004366067
(71) Applicant: Shiseido Company, Limited, Chuo-ku Tokyo 104-8010 (JP)
(72) Inventor: MAEDA, Kazuhisa, SHISEIDO RES. CEN.(SHIN-YOKOHAMA), Yokohama-shi, Kanagawa, 224-8558 (JP); NAWAMURA, Takeshi, SHISEIDO RES CEN. SHIN-YOKOHAMA, Yokohama-shi, Kanagawa, 224-8558 (JP); KOBAYASHI, Koji, SHISEIDO RES. CEN.(SHIN-YOKOHAMA), Yokohama-shi, Kanagawa, 224-8558 (JP); CHIN, Nuan, SHISEIDO CHINA RES. CEN. CO. LTD., Bejin Ec Tech Dev. Zone, Beijing 100176 (CN)
(74) Representative: Ulmann, Catherine Claire
(86) International application number: PCT/JP2005/023552
(87) International publication number: WO 2006/064974

(57) **Abstract**

An external preparation for skin whitening containing 0.001 to 20.0 mass% of an extract of at least one plant selected from the group consisting of *Pilea cavaleriei* (Urticaceae), *Saururus chinensis* (Saururaceae), *Streptocaulon griffithii* (Asclepiadaceae), *Toddalia asiatica* (Rutaceae) and *Tadehagi triquetrum* (Leguminosae).

## Description

### TECHNICAL FIELD

The present invention relates to a novel whitening agent and an external preparation for skin whitening containing the same, more particularly relates to an external preparation for skin whitening effective for suppressing the formation of melanin and for the prevention and alleviation of blemishes, freckles and other pigmentation.

### BACKGROUND ART

Blemishes, freckles and other pigmentation are considered to be caused by hormonal abnormalities, ultraviolet light and topical inflammation of the skin leading to melanin being excessively formed and depositing in the skin. The melanin causing pigmentation of the skin is produced in organelles called melanosomes within melanocytes at the basal epithelial layer. The melanin thus produced is incorporated into the keratinocytes. The melanin in the melanocytes are produced by changing tyrosine to black melanin by the action of the enzyme tyrosinase through dopaquinone by an enzymatic or nonenzymatic oxidation reaction. Therefore, the suppression of the first stage reaction, that is, the activity of tyrosinase, is important in suppressing the production of melanin.

Substances having a whitening effect, that is, substances suppressing the production of melanin, are mainly used for the purpose of the prevention and alleviation of such pigment abnormalities. The method of orally administering a large amount of vitamin C, the method of injecting glutathione and the like, the method of topical application of Kojic acid, vitamin C or its derivative, cysteine and the like in the form of an ointment, cream, lotion and the like, etc. are known.

However, compounds suppressing the activity of tyrosinase, other than hydroquinone, are extremely mild in the manifestation of the effect, so the effect of the improvement of skin pigmentation is insufficient. On the other hand, hydroquinone has a recognizable effect, but has sensitivity, so the general use thereof is restricted. Therefore, to improve the safety, attempts have been made for use of monoesters of higher fatty acids or alkyl monoethers (Japanese Patent Publication No. 58-154507A).

### DISCLOSURE OF THE INVENTION

However, the monoesters of higher fatty acids are not necessarily safe, since they are decomposed by hydrolase in the body. Further, the ethers are also not sufficiently satisfactory in terms of the safety.

Accordingly, the present inventors investigated the effect of various plant extracts on melanin production and, as a result, found that specific plant extracts not previously known to have this effect have an action in suppressing melanin production and an action in inhibiting tyrosinase, whereby the present invention was completed.

In accordance with the present invention, there is provided an external preparation for skin whitening comprising 0.001 to 20.0 mass% of an extract of at least one plant selected from the group consisting of *Pilea cavaleriei* of plants belonging to the *Urticaceae family, Saururus chinensis* of plants belonging to the Saururaceae *family, Streptocaulon griffithii* of plants belonging to the *Asclepiadaceae family, Toddalia asiatica* of plants belonging to the *Rutaceae family* and *Tadehagi triquetrum* of plants belonging to the *Leguminosae family,* and a base ingredient.

In accordance with the present invention, there is also provided a whitening agent comprising an extract of at least one plant selected from the group consisting of *Pilea cavaleriei* of plants belonging to the *Urticaceae family, Saururus chinensis* of plants belonging to the Saururaceae *family, Streptocaulon griffithii* of plants belonging to the *Asclepiadaceae family, Toddalia asiatica* of plants belonging to the *Rutaceae family* and *Tadehagi triquetrum* of plants belonging to the *Leguminosae family.*

In accordance with the present invention, there is further provided a whitening method using the above whitening agent for whitening the skin.

In accordance with the present invention, there is still further provided a method for producing an external preparation for skin whitening producing an external preparation for skin whitening by formulating the above whitening agent into an aqueous phase or an oil phase.

The *Pilea cavaleriei* used by the present invention is known to be used in China as a folk medicine for lung tuberculosis, pneumonic cough, edema due to kidney inflammation, traumatic swelling and pain, burns, scalding, acne, small growths and the like.

*Saururus chinensis* is called "lizard's tail" and is known to be used as a diuretic.

*Streptocaulon griffithii* is known to be used for the fever of colds, cholera, acute gastroenteritis, dysentery, the pain of gastrocardiac weakness, and the like.

*Toddalia asiatica* is called "Sarukake-mikan" in Japanese and is known to be used for cold, hematemesis, cough, stomach ache, the numbness and pain of rheumatism and the like.

*Tadehagi triquetrum* is called "Tadehagi" and is known to be used for the fever of colds, hepatitis, kidney inflammation, childhood dyspepsia, pregnancy, vomiting, heat and humidity-based diarrhea, cutaneous ulcers, boils and the like.

An external preparation for skin whitening according to the present invention has an action in suppressing melanin production, has a superior effect in lightening and whitening pigmentation after sunburn, blemishes, freckles, liver spots and the like, and is superior in safety.

According to the whitening agent of the present invention, it is possible to provide an external preparation for skin whitening which uses the whitening agent to obtain an action in suppressing melanin production, has a superior effect in lightening and whitening pigmentation after sunburn, blemishes, freckles, liver spots and the like, and is superior in safety.

Further, according to the present invention, there is provided a whitening method having a superior effect in lightening and whitening pigmentation after sunburn, blemishes, freckles, liver spots and the like and is superior in safety.

Furthermore, according to the production method of an external preparation for skin whitening according to the present invention, it is possible to produce an external preparation for skin whitening having an action in suppressing melanin production, having a superior effect in lightening and whitening pigmentation after sunburn, blemishes, freckles, liver spots and the like, and superior in safety.

### BEST MODE FOR CARRYING OUT THE INVENTION

The constitution of the present invention will now be explained in detail.

Terms in the singular form used herein shall include the plural, except when clearly singular from the context.

The *Pilea cavaleriei, Saururus chinensis, Streptocaulon griffithii, Toddalia asiatica* and *Tadehagi triquetrum* used for the whitening agent of the present invention are plants particular growing in China, Japan, Southeast Asia, etc.

The plant extract used in the present invention is obtained by immersing or heating and refluxing the plant leaves, the stems including the rhizome, root, fruit, whole plant, etc. together with an extracting solvent, then filtrating and concentrating the result and mixture. As the extraction solvent in the present invention, any solvent commonly used in extraction may be usable. Particularly, alcohols such as methanol, ethanol, aqueous alcohols, and organic solvents such as acetone, ethyl acetate can be used alone or in any combination thereof. The whitening agent of the present invention comprises the above plant extract, but may either be a plant extract from the above plant alone or a plant extract from mixed plants.

The amount of the above plant extract in the external preparation for skin whitening according to the present invention is 0.001 to 20.0 mass%, preferably 0.01 to 10.0 mass%, as a dried substance, in the total mass of the external preparation. If the amount is less than 0.001 mass%, the effect of the present invention tends to becomes poorer, while if the amount is more than 20.0 mass%, formation into a preparation is difficult, so these are not desirable. Further, even if mixing in more than 10.0 mass%, the further improvement in the effect can not be seen, as expected.

The external preparation for skin whitening according to the present invention may suitably and optionally contain, other than the above stated essential ingredients, ingredients used for external use skin agents of usual cosmetics, pharmaceuticals and the like, for example, base ingredients such as other whitening agents, moisturizing agents, antioxidants, oil ingredients, ultraviolet absorbents, surfactants, thickening agents, alcohols, powder ingredients, coloring materials, aqueous ingredients, water, various skin nutrients.

In addition, sequestering agents such as disodium edetate, trisodium edetate, sodium citrate, sodium polyphosphate, sodium metaphosphate, gluconic acid, medicines such as caffeine, tannin, verapamil, tranexamic acid and their derivatives, licorice extract, glabridin, hot water extracts of the fruit of firethorn, various herbal medicines, tocopherol acetate, glycyrrhizic acid and their derivatives or their salts, other whitening agents, such as vitamin C, magnesium ascorbic acid phosphate, glucoside ascorbate, arbutin, Kojic acid, rucinol, ellagic acid, chamomilla, saccharides such as glucose, fructose, mannose, sucrose, trehalose may be suitably contained.

The "external preparation for skin whitening" of the present invention, for example, may be in the form of any of an ointment, cream, emulsion, lotion, pack, bath agent and other preparations used for external use. The form is not particularly limited.

### EXAMPLES

In the following, the present invention is further described in detail by Examples. Note that the present invention is not limited to these Examples. The amounts are mass%. Before the Examples, the effect in the suppression of melanin production of the plant extracts of the present invention, the test methods relating to the whitening effect, and the results will be explained.

### Test Methods and Results

### 1. Preparation of Test Samples

### (1) Pilea cavaleriei extract

60 g of the dried whole plant of *Pilea cavaleriei* was immersed in methanol at room temperature for 1 week. The methanol extract was filtered, and the solvent was removed by evaporation. Finally, 1.6 g of *Pilea cavaleriei* methanol extract was obtained. Then, the extract was dissolved in DMSO at 1 mass%, and the solution was used as a test sample in the following experiments at appropriate dilution.

### (2) Saururus chinensis extract

31 g of the dried root and rhizome of *Saururus chinensis* was immersed in methanol at room temperature for 1 week. The methanol extract was filtered, and the solvent was removed by evaporation. Finally, 1.6 g of *Saururus chinensis* methanol extract was obtained. Then, the extract was dissolved in DMSO at 1 mass%, and the solution was used as a test sample in the following experiments at appropriate dilution.

### (3) Streptocaulon griffithii extract

30 g of the dried root of *Streptocaulon griffithii* was immersed in methanol at room temperature for 1 week. The methanol extract was filtered, and the solvent was removed by evaporation. Finally, 1.4 g of *Streptocaulon griffithii* methanol extract was obtained. Then, the extract was dissolved in DMSO at 1 mass%, and the solution was used as a test sample in the following experiments at appropriate dilution.

### (4) Toddalia asiatica extract

33 g of the dried root of *Toddalia asiatica* was immersed in methanol at room temperature for 1 week. The methanol extract was filtered, and the solvent was removed by evaporation. Finally, 2.9 g of *Toddalia asiatica* methanol extract was obtained. Then, the extract was dissolved in DMSO at 1 mass%, and the solution was used as a test sample in the following experiments at appropriate dilution.

### (5) Tadehagi triquetrum extract

30 g of the dried whole plant of *Tadehagi triquetrum* was immersed in methanol at room temperature for 1 week. The methanol extract was filtered, and the solvent was removed by evaporation. Finally, 3.0 g of *Tadehagi triquetrum* methanol extract was obtained. Then, the extract was dissolved in DMSO at 1 mass%, and the solution was used as a test sample in the following experiments at appropriate dilution.

### 2. Cell Culture Method

Mouse B16 melanoma cells were used. These were cultured in Eagle's MEM containing 10% FBS and theophylline (0.09 mg/ml) in a CO₂ incubator (95% air, 5% carbon dioxide) at 37°C. After 24 hours of culture, the sample solution was added to give a final concentration of 2×10⁻³ to 2×10⁻⁵ weight% (as dried extract concentration). After further incubation for 3 days, the amount of melanin was determined visually by the following method.

### 3. Visual Determination of Melanin Amount

A diffuser plate was placed on the lid of a well plate, the number of cells and the amount of melanin in the cells were observed by an inverted microscope and compared with a reference sample to which no plant extracts were added. The results are shown in Table I. Further, as a positive control, a similar examination as the above was performed for arbutin, which is already known to have an action suppressing melanin production. No suppressive effect on cell proliferation was observed in each sample.

### Judgment Criteria

Good: whiter in comparison with the reference, and determined that the amount of melanin was small.
Fair: slightly whiter in comparison with the reference, and determined that the amount of melanin was slightly small.
Poor: equally white in comparison with the reference, and determined that the amount of melanin was almost the same.

**Table I**

| Sample | Visual Evaluation of Melanin Production | | | | |
|---|---|---|---|---|---|
| Concentration (mass%) | 0.000001 | 0.00025 | 0.0005 | 0.001 | 0.002 |
| *Pilea cavaleriei* | - | - | - | Fair | Good |
| *Saururus chinensis* | Good | - | - | - | - |
| *Streptocaulon griffithii* | - | Fair | Good | - | - |
| *Toddalia asiatica* | - | - | - | Fair | Good |
| *Tadehagi triquetrum* | - | - | - | Fair | Good |
| Arbutin | - | - | - | Fair | Good |

As shown in Table I, the extracts of Pilea cavaleriei, Saururus chinensis, Streptocaulon griffithii, Toddalia asiatica, and Tadehagi triquetrum have a suppressive effect on the melanin production, without affecting cell proliferation, and it is learned that they have a superior whitening effect similar to arbutin.

### 4. Whitening Effect Test

### (4-1) Preparation of samples for external skin use

Samples were prepared by the following formula. The preparations were made by combining the alcohol phase and the aqueous phase.

| (Alcohol Phase) | |
|---|---|
| 99% Ethanol | 70.0 mass% |
| Whitening agent of Table II | Amount of Table II |

| (Aqueous Phase) | |
|---|---|
| Glycerin | 5.0 |
| Ion exchanged water | Balance |

### (4-2) Test Method

The panels' (n=5) skin were exposed to ultraviolet light. From the 14th day after the exposure, the formulations were applied to the skins once a day for 8 weeks. After the 8 weeks application, it was investigated whether there was an effect of suppression on pigmentation induced by UV irradiation, by four levels of evaluation criteria. The results are shown in Table II.

### (Evaluation Criteria)

4: Remarkably effective
3: Effective
2: Somewhat effective
1: No effect

**Table II**

| Sample | Amount (mass%) | Effect |
|---|---|---|
| No addition | - | 1 1 1 1 1 |
| *Pilea cavaleriei* | 0.5 | 2 2 2 1 2 |
| *Saururus chinensis* | 0.5 | 3 2 2 2 3 |
| *Streptocaulon griffithii* | 0.5 | 3 1 3 2 2 |
| *Toddalia asiatica* | 0.5 | 2 1 2 2 2 |
| *Tadehagi triquetrum* | 0.5 | 1 2 1 2 2 |

As is clear from Table II, formulations containing *Pilea cavaleriei, Saururus chinensis, Streptocaulon griffithii, Toddalia asiatica* or *Tadehagi triquetrum* were recognized to prevent the deposition of melanin pigment and to prevent or alleviate the blackening on the UV-exposed panel skin.

Formulation Examples of external preparations for skin according to the present invention are shown in the following. The whitening agents (plant extracts) were prepared by the same method as described above. The amounts are shown by mass%. The external use skin agents obtained by Examples 1 to 10 were all recognized as being effective in the whitening effect test.

**Example 1: Cream**

| (Formulation) | (Mass%) |
|---|---|
| Stearic acid | 5.0 |
| Stearyl alcohol | 4.0 |
| Isopropyl myristate | 18.0 |
| Glyceryl monostearic acid ester | 3.0 |
| Propylene glycol | 10.0 |
| *Pilea cavaleriei* ethanol extract | 0.01 |
| Potassium hydroxide | 0.2 |
| Sodium hydrogen sulfite | 0.01 |
| Preservative | q.s. |
| Perfume | q.s. |
| Ion exchanged water | Bal. |

### (Process of Production)

The propylene glycol, *Pilea cavaleriei* ethanol extract and potassium hydroxide were added to and dissolved in the ion exchanged water and heated to and held at 70°C (aqueous phase). The rest of the ingredients were mixed, heated to melt, and held at 70°C (oil phase). The oil phase was gradually added to the aqueous phase. After the addition of the entire amount was finished, the temperature was held there for a while to cause a reaction. Thereafter, the mixture was uniformly emulsified by a homo mixer and cooled to 30°C while vigorously stirring.

**Example 2: Cream**

| (Formulation) | (Mass%) |
|---|---|
| Stearic acid | 2.0 |
| Stearyl alcohol | 7.0 |
| Hydrogenated lanolin | 2.0 |
| Squalane | 5.0 |
| 2-octyldodecyl alcohol | 6.0 |
| Polyoxyethylene (25 mol) cetyl alcohol ether | 3.0 |
| Glyceryl monostearic acid ester | 2.0 |
| Propylene glycol | 5.0 |
| *Saururus chinensis* hexane extract | 0.05 |
| Sodium hydrogen sulfite | 0.03 |
| Ethylparaben | 0.3 |
| Perfume | q.s. |
| Ion exchanged water | Bal. |

### (Process of Production)

Propylene glycol was added to the ion exchanged water and heated to and held at 70°C (aqueous phase). The rest of the ingredients were mixed, heated to melt, and held at 70°C (oil phase). The oil phase was added to the aqueous phase and preemulsified, was uniformly emulsified by a homo mixer, then was cooled to 30°C while vigorously stirring.

**Example 3: Cream**

| (Formulation) | (Mass%) |
|---|---|
| Solid paraffin | 5.0 |
| Beeswax | 10.0 |
| Petrolactum | 15.0 |
| Liquid paraffin | 41.0 |
| Glyceryl monostearic acid ester | 2.0 |
| Polyoxyethylene (20 mol) sorbitan monolauric acid ester | 2.0 |
| Soap powder | 0.1 |
| Borax | 0.2 |
| *Streptocaulon griffithii* acetone extract | 0.05 |
| Sodium hydrogen sulfite | 0.03 |
| Ethylparaben | 0.3 |
| Perfume | q.s. |
| Ion exchanged water | Bal. |

### (Process of Production)

The soap powder and borax were added to the ion exchanged water, heated to dissolve, and held at 70°C (aqueous phase). The rest of the ingredients were mixed, heated to melt, and held at 70°C (oil phase). The oil phase was gradually added to the aqueous phase while stirring to cause a reaction. After the end of the reaction, the resultant mixture was uniformly emulsified by a homo mixer and cooled to 30°C while vigorously stirring after emulsification.

**Example 4: Milky Lotion**

| (Formulation) | (Mass%) |
|---|---|
| Stearic acid | 2.5 |
| Cetyl alcohol | 1.5 |
| Petrolactum | 5.0 |
| Liquid paraffin | 10.0 |
| Polyoxyethylene (10 mol) monooleic acid ester | 2.0 |
| Polyethylene glycol 1500 | 3.0 |
| Triethanol amine | 1.0 |
| Carboxyvinyl polymer (Name of product: Carbopol 941, B.F. Goodrich Chemical Company) | 0.05 |
| *Toddalia asiatica* ethyl acetate extract | 0.01 |
| Sodium hydrogen sulfite | 0.01 |
| Ethylparaben | 0.3 |
| Perfume | q.s. |
| Ion exchanged water | Bal. |

### (Process of Production)

The carboxyvinyl polymer was dissolved in a small amount of ion exchanged water (phase A). The polyethylene glycol 1500 and triethanol amine were added to the remaining ion exchanged water, heated to dissolve, and held at 70°C (aqueous phase). The rest of the ingredients were mixed, heated to melt, and held at 70°C (oil phase). The oil phase was added to the aqueous phase, the mixture was preemulsified, the phase A was added, then the result was uniformly emulsified by a homo mixer and cooled to 30°C while vigorously stirring after emulsification.

**Example 5: Milky Lotion**

| (Formulation) | (Mass%) |
|---|---|
| Microcrystalline wax | 1.0 |
| Beeswax | 2.0 |
| Lanolin | 20.0 |
| Liquid paraffin | 10.0 |
| Squalane | 5.0 |
| Sorbitan sesquioleic acid ester | 4.0 |
| Polyoxyethylene (20 mol) sorbitan monooleic acid ester | 1.0 |
| Propylene glycol | 7.0 |
| *Tadehagi triquetrum* water extract | 10.0 |
| Sodium hydrogen sulfite | 0.01 |
| Ethylparaben | 0.3 |
| Perfume | q.s. |
| Ion exchanged water | Bal. |

### (Process of Production)

The propylene glycol was added to the ion exchanged water, heated, and held at 70°C (aqueous phase). The rest of the ingredients were mixed, heat to melt, and held at 70°C (oil phase). While stirring the oil phase, the aqueous phase was gradually added and the mixture was uniformly emulsified by a homo mixer. This was then cooled to 30°C, while vigorously stirring after emulsification.

**Example 6: Gel**

| (Formulation) | (Mass%) |
|---|---|
| 95% ethyl alcohol | 10.0 |
| Dipropylene glycol | 15.0 |
| Polyoxyethylene (50 mol) oleyl alcohol ether | 2.0 |
| Carboxyvinyl polymer (Name of Product: Carbopol 940, B.F. Goodrich Chemical Company) | 1.0 |
| Caustic soda | 0.15 |
| L-arginine | 0.1 |
| *Pilea cavaleriei* 50% ethanol (ethanol/water) extract | 7.0 |
| Sodium 2-hydroxy-4-methoxybenzophenone sulfonate | 0.05 |
| Ethylenediamine tetraacetate. 3 sodium·2 water | 0.05 |
| Methylparaben | 0.2 |
| Perfume | q.s. |
| Ion exchanged water | Bal. |

### (Process of Production)

The Carbopol 940 was uniformly dissolved in the ion exchanged water. On the other hand, the *Pilea cavaleriei* 50% ethanol (ethanol/water) extract and polyoxyethylene (50 mol) oleyl alcohol ether were dissolved in the 95% ethanol, and the resultant mixture was added to the aqueous phase. Next, the rest of the ingredients were added, then the result was neutralized and thickened with the caustic soda and L-arginine.

**Example 7: Essence**

| (Formulation) | (Mass%) |
|---|---|
| (Phase A) | |
| Ethyl alcohol (95%) | 10.0 |
| Polyoxyethylene (20 mol) octyldodecanol | 1.0 |
| Pantothenyl ethyl ether | 0.1 |
| *Saururus chinensis* ethanol extract | 1.5 |
| Methylparaben | 0.15 |
| (Phase B) | |
| Potassium hydroxide | 0.1 |
| (Phase C) | |
| Glycerin | 5.0 |
| Dipropylene glycol | 10.0 |
| Sodium hydrogen sulfite | 0.03 |
| Carboxyvinyl polymer (Name of Product: Carbopol 940, B.F. Goodrich Chemical Company) | 0.2 |
| Purified water | Bal. |

### (Process of Production)

The phase A and phase C were uniformly dissolved, then phase A was added to the phase C to solubilize it. Next, the phase B was added, then the resultant mixture was filled.

**Example 8: Pack**

| (Formulation) | (Mass%) |
|---|---|
| (Phase A) | |
| Dipropylene glycol | 5.0 |
| Polyoxyethylene (60 mol) hydrogenated castor oil | 5.0 |

| (Phase B) | |
|---|---|
| *Streptocaulon griffithii* ethanol extract | 0.01 |
| Olive oil | 5.0 |
| Tocopherol acetate | 0.2 |
| Ethylparaben | 0.2 |
| Perfume | 0.2 |

| (Phase C) | |
|---|---|
| Sodium hydrogen sulfite | 0.03 |
| Polyvinyl alcohol (saponification degree 90, polymerization degree 2000) | 13.0 |
| Ethanol | 7.0 |
| Purified water | Bal. |

### (Process of Production)

The phase A, phase B and phase C were uniformly dissolved, phase B was added to the phase A to solubilize it. Next, the phase C was added thereto, then the resultant mixture was filled.

**Example 9: Solid Foundation**

| (Formulation) | (Mass%) |
|---|---|
| Talc | 43.1 |
| Kaolin | 15.0 |
| Sericite | 10.0 |
| Zinc oxide | 7.0 |
| Titanium dioxide | 3.8 |
| Yellow iron oxide | 2.9 |
| Black iron oxide | 0.2 |
| Squalane | 8.0 |
| Isostearic acid | 4.0 |
| Sorbitan monooleate POE | 3.0 |
| Isocetyl octanoate | 2.0 |
| *Toddalia asiatica* ethanol extract | 1.0 |
| Preservative | q.s. |
| Perfume | q.s. |

### (Process of Production)

The powder ingredients of the talc to the black iron oxide were sufficiently mixed by a blender, the oil ingredients of the squalane to isocetyl octanoate, the *Toddalia asiatica* ethanol extract, the preservative and the fragrance were added thereto, then the resultant mixture was filled into a container and shaped.

**Example 10: Emulsified Foundation (Cream Type)**

| (Formulation) | (Mass%) |
|---|---|
| (Powder part) | |
| Titanium dioxide | 10.3 |
| Sericite | 5.4 |
| Kaolin | 3.0 |
| Yellow iron oxide | 0.8 |
| Bengara | 0.3 |
| Black iron oxide | 0.2 |

| (Oil phase) | |
|---|---|
| Decamethyl cyclopentasiloxane | 11.5 |
| Liquid paraffin | 4.5 |
| Polyoxyethylene-modified | |
| dimethyl polysiloxane | 4.0 |

| (Aqueous phase) | |
|---|---|
| Purified water | 50.0 |
| 1,3-butylene glycol | 4.5 |
| *Tadehagi triquetrum* ethanol extract | 1.5 |
| Sorbitan sesquioleate ester | 3.0 |
| Preservative | q.s. |
| Perfume | q.s. |

### (Process of Production)

The aqueous phase was heated and stirred, then the sufficiently mixed and crushed powder part was added and treated by a homo mixer. Further, the heated and mixed oil phase was added, the resultant mixture treated by a homo mixer, then the fragrance was added and the resultant mixture cooled to room temperature, while stirring.

## Claims

1. An external preparation for skin whitening **characterized by** comprising 0.001 to 20.0 mass%, as a dried substance, of an extract of at least one plant selected from the group consisting of *Pilea cavaleriei* of plants belonging to the *Urticaceae family, Saururus chinensis* of plants belonging to the *Saururaceae family, Streptocaulon griffithii* of plants belonging to the *Asclepiadaceae family, Toddalia asiatica* of plants belonging to the *Rutaceae family* and *Tadehagi triquetrum* of plants belonging to the *Leguminosa family* e and a base ingredient.

2. A whitening agent **characterized by** comprising an extract of at least one plant selected from the group consisting of *Pilea cavaleriei* of plants belonging to the *Urticaceae family, Saururus chinensis* of plants of the *Saururaceae family, Streptocaulon griffithii* of plants belonging to the *Asclepiadaceae family, Toddalia asiatica* of plants belonging to the *Rutaceae family* and *Tadehagi triquetrum* of plants belonging to the *Leguminosae family.*

3. A whitening method **characterized by** using the whitening agent according to claim 2 for whitening the skin.

4. A method for producing an external preparation for skin whitening **characterized by** producing an external preparation for skin whitening by formulating the whitening agent according to claim 2 into an aqueous phase or an oil phase.
